(19) Europäisches Patentamt
European Patent Office
Office européen des brevets

(11) Publication number: 0 153 803
A2

# (12) EUROPEAN PATENT APPLICATION

(21) Application number: 85300401.8

(22) Date of filing: 22.01.85

(51) Int. Cl.[4]: C 07 D 249/08
A 61 K 31/41, A 01 N 43/653

(30) Priority: 24.01.84 GB 8401738
14.08.84 GB 8420583

(43) Date of publication of application:
04.09.85 Bulletin 85/36

(84) Designated Contracting States:
AT BE CH DE FR GB IT LI LU NL SE

(71) Applicant: Pfizer Limited
Ramsgate Road
Sandwich Kent CT13 9NJ(GB)

(84) Designated Contracting States:
GB

(71) Applicant: Pfizer Corporation
Calle 15 1/2 Avenida Santa Isabel
Colon(PA)

(84) Designated Contracting States:
BE CH DE FR IT LI LU NL SE AT

(72) Inventor: Richardson, Kenneth, Dr.
48 St. Stephens Hill
Canterburry Kent(GB)

(72) Inventor: Gymer, Geoffrey Edward, Dr.
19 Palmer Road
Wingham Canterbury(GB)

(74) Representative: Wood, David John et al,
Pfizer Limited Ramsgate Road
Sandwich Kent CT13 9NJ(GB)

## (54) Triazole antifungal agents.

(57) A triazole of the general formula:

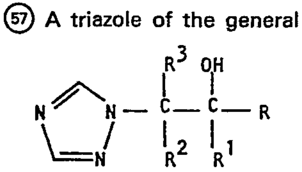

--- (1)

or an O-ester, O-ether or pharmaceutically or agriculturally acceptable salt thereof,
wherein

R and $R^1$ are each independently a phenyl group optionally substituted by 1 to 3 substituents each independently selected from F, C1, Br, I, $CF_3$, $C_1$-$C_4$ alkyl and $C_1$-$C_4$ alkoxy;

$R^2$ is H, $CH_3$ or F; and

$R^3$ is H or $CH_3$;

with the proviso that one of $R^2$ and $R^3$ is other than H.

The compounds are useful as human and agricultural antifungal agents.

EP 0 153 803 A2

This invention relates to novel triazole derivatives which have antifungal activity and are useful in the treatment of fungal infections in animals, including humans, and as agricultural (including horticultural) fungicides.

According to the invention, there are provided compounds of the formula:-

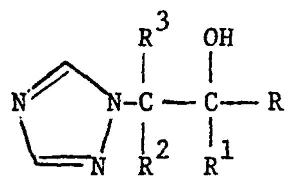

--- (I)

and their O-esters, O-ethers and pharmaceutically and agriculturally acceptable salts,

where R and $R^1$ are each independently a phenyl group optionally substituted by 1 to 3 substituents each independently selected from F, Cl, Br, I, $CF_3$, $C_1$-$C_4$ alkyl and $C_1$-$C_4$ alkoxy,

$R^2$ is H, $CH_3$ or F;

and $R^3$ is H or $CH_3$;

with the proviso that one of $R^2$ and $R^3$ is other than H.

The invention further provides a compound of the formula (I) or an O-ester, O-ether or pharmaceutically acceptable salt thereof, for use in medicine, in particular for treating a fungal infection in a human being.

The invention further includes a fungicidal composition for human or agricultural use, comprising a compound of the formula (I), or an O-ester, O-ether or pharmaceutically or agriculturally acceptable salt thereof, together with a pharmaceutically or agriculturally acceptable diluent or carrier.

It also provides a method of treating a human being having a fungal infection, which comprises administering to said human an effective amount of a compound of the formula (I) or O-ester, O-ether or pharmaceutically acceptable salt thereof.

The invention also includes a method of treating or preventing a fungal infection on seeds or plants, which comprises contacting said seeds or plants or the locus thereof, with an antifungally effective amount of a compound of the formula (I) or O-ester, O-ether or agriculturally acceptable salt thereof.

R and $R^1$ are each preferably phenyl substituted by 1 to 3 substituents, more preferably 1 or 2 substituents, each independently selected from F, Cl, Br, I and $CF_3$. In particular in this aspect, R and $R^1$ can each be 4-fluorophenyl, 4-chlorophenyl, 4-trifluoromethylphenyl, 2-fluorophenyl, 2,4-dichlorophenyl, 2,4-difluorophenyl, 4-chloro-2-fluorophenyl, 2-chloro-4-fluorophenyl, 2,5-difluorophenyl, 2,4,6-trifluorophenyl or 4-bromo-2,5-difluorophenyl. Most preferably, R and $R^1$ are each 4-chlorophenyl, 4-fluorophenyl, 2,4-dichlorophenyl or 2,4-difluorophenyl.

In one aspect of the invention $R^2$ is F and $R^3$ is H, and in another aspect $R^2$ is H, $CH_3$ or F and $R^3$ is $CH_3$.

The O-ethers of the alcohols of the formula (I) include, for example, the $C_1$-$C_6$ alkyl, $C_2$-$C_4$ alkenyl, $C_2$-$C_4$ alkynyl and aralkyl (e.g. benzyl optionally ring-substituted by 1 or 2 substituents each selected from halo, $C_1$-$C_4$ alkyl and $C_1$-$C_4$ alkoxy) ethers.

The O-esters of the alcohols of the formula (I) include, for example, the $C_2$-$C_4$ alkanoyl and aroyl (e.g. benzoyl, optionally

substituted by 1 or 2 substituents each independently selected from halo, $C_1$-$C_4$ alkyl and $C_1$-$C_4$ alkoxy) esters.

The preferred O-ester is the acetyl ester.

"Halo" means F, Cl, Br or I.

The compounds of the formula (I) can be prepared according to the following reaction scheme:-

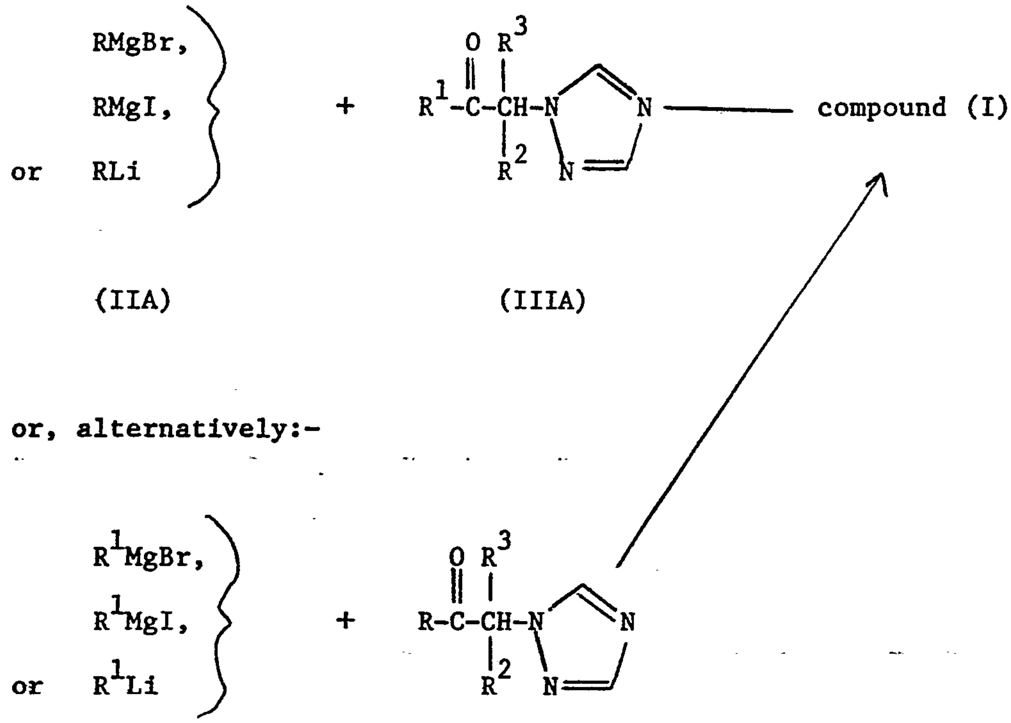


(IIA) (IIIA)

or, alternatively:-

(IIB) (IIIB)

In a typical reaction, a solution of the organometallic compound (IIA or B) in a suitable organic solvent, e.g. dry diethyl ether, is added dropwise to a solution of the ketone (IIIA or B) in e.g. dry diethyl ether at low temperature, preferably at about -70°C and under nitrogen. Tetrahydrofuran is an alternative solvent. The reaction mixture is then allowed to warm to room temperature. If necessary, the reaction mixture can be heated,

e.g. under reflux at up to 70°C maximum, until the reaction is complete, e.g. in a few hours. The product (I) can then be recovered and purified conventionally. When the product (I) has one or two chiral centres, the optical isomers or diastereomeric pairs can be separated conventionally.

The ketonic starting materials (IIIA) and (IIIB) can be prepared by conventional methods such as are described in the following Preparations.

For example, the ketones in which $R^3$ is $CH_3$ and $R^2$ is H or $CH_3$ can be prepared by methylation under conventional conditions (see e.g. European patent applications publication nos. 0122056 and 0120276) as follows:-

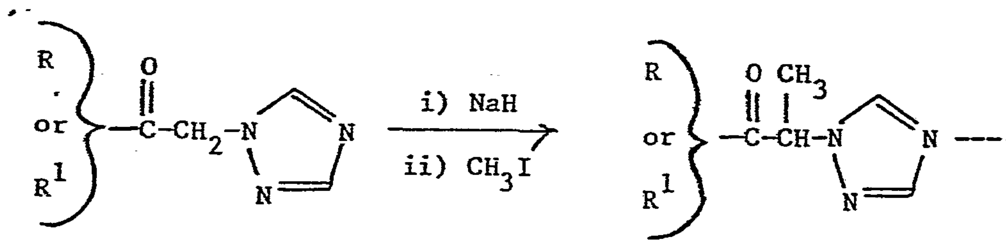


(IV)

(IIIC)

or

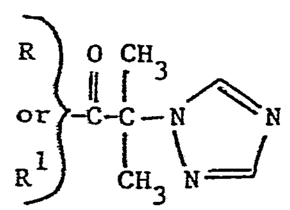

(IIID)

(if 2 equivalents of $NaH/CH_3I$ used)

The starting materials (IV) are known compounds (see e.g. European patent application publication no. 0044605 or UK patent specifications nos. 2099818A, 1464224, 1533705 and 1533706).

The fluoro-substituted ketones (IIIA and B, $R^2 = F$) can in turn be prepared by the fluorination of the ketones (IV) or (IIIC) using sodium hydride followed by perchloryl fluoride. An alternative route (see Preparation 6) involves the reaction of the appropriate 2-chloro-2-fluoroacetophenone with 1,2,4-triazole in the presence of potassium carbonate.

When R and $R^1$ are the same and $R^2$ and $R^3$ are $CH_3$, the following method can also be used to prepare the end products:-

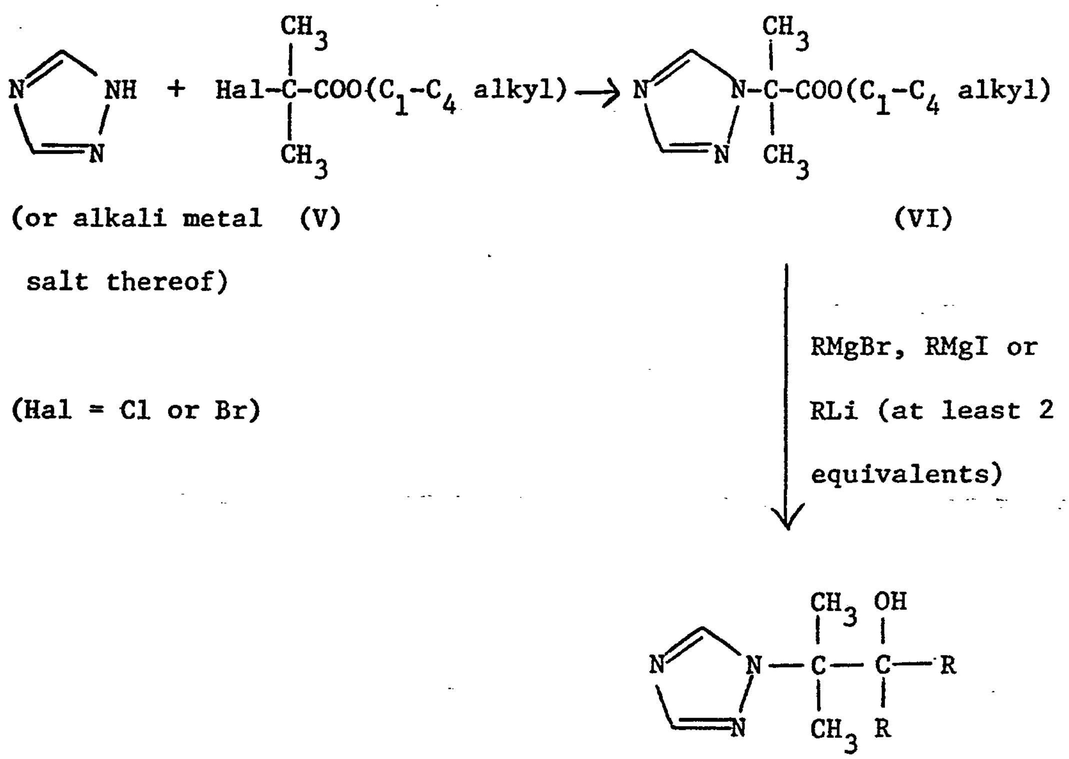


(or alkali metal salt thereof) (V) (VI)

(Hal = Cl or Br)

(IA).

Typically, sodium hydride in a suitable organic solvent, e.g. dry dimethylformamide (DMF), has 1,2,4-triazole in the same solvent added to it at about 0°C. The ester (V), preferably as ethyl 2-bromoisobutyrate, is then added in DMF, and after the initial exothermic reaction has subsided, the reaction mixture is heated, at, say, 50-80°, until conversion to the intermediate (VI) is sufficiently complete. After purification, the intermediate (VI) is reacted with the Grignard or lithio reagent RMgBr, RMgI or RLi in a similar manner to the previous route, although it is of course necessary to have at least two equivalents of the said reagent present. The product (IA) can then be isolated and purified in a conventional manner.

The ethers can be made by treating an alkali metal salt of the alcohols of the formula (I), e.g. a lithium or sodium salt, with the appropriate halide, e.g. an alkyl, alkenyl, alkynyl, aryl or aralkyl halide. Esters can be made by treating an alkali metal salt of compound (I) with the appropriate acid chloride, bromide or anhydride.

Where the compounds of the invention contain one or more chiral centres, the invention includes both the resolved and unresolved forms. Example 7 illustrates the separation of a product of the formula (I) into its two diastereomeric pairs, and Example 12 illustrates the resolution of a product into its two optically active forms.

Pharmaceutically and agriculturally acceptable acid addition salts of the compounds of the formula (I) are those formed from strong acids which form non-toxic acid addition salts, such as hydrochloric, hydrobromic, sulphuric, oxalic and methanesulphonic acids.

The salts may be obtained by conventional procedures, e.g. by mixing solutions containing equimolar amounts of the free base and desired acid, and the required salt is collected by filtration, if insoluble, or by evaporation of the solvent.

Also included are the alkali metal salts, preparable conventionally.

The compounds of the formula (I) including their O-esters, O-ethers and pharmaceutically acceptable salts are antifungal agents, useful in combating fungal infections in animals, including humans. For example they are useful in treating topical fungal infections in man caused by, among other organisms, species of *Candida*, *Trichophyton*, *Microsporum* or *Epidermophyton*, or in mucosal infections caused by *Candida albicans* (e.g. thrush and vaginal candidiasis). They can also be used in the treatment of systemic fungal infections caused by, for example, *Candida albicans*, *Cryptococcus neoformans*, *Aspergillus fumigatus*, *Coccidioides*, *Paracoccidioides*, *Histoplasma* or *Blastomyces*.

The *in vitro* evaluation of the antifungal activity of the compounds can be performed by determining the minimum inhibitory concentration (m.i.c.) which is the concentration of the test compounds in a suitable medium at which growth of the particular micro-organism fails to occur. In practice, a series of agar

plates, each having the test compound incorporated at a particular concentration is inoculated with a standard culture of, for example, Candida albicans and each plate is then incubated for 48 hours at 37°C. The plates are then examined for the presence or absence of growth of the fungus and the appropriate m.i.c. value is noted. Other micro-organisms used in such tests can include Cryptococcus neoformans, Aspergillus fumigatus, Trichophyton spp; Microsporum spp; Epidermophyton floccosum, Coccidioides immitis and Torulopsis glabrata.

The in vivo evaluation of the compounds can be carried out at a series of dose levels by intraperitoneal or intravenous injection or by oral administration, to mice which are inoculated with a strain of Candida albicans. Activity is based on the survival of a treated group of mice after the death of an untreated group of mice following 48 hours observation. The dose level at which the compound provides 50% protection ($PD_{50}$) against the lethal effect of the infection is noted.

For human use, the antifungal compounds of the formula (I) can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules or ovules either alone or in admixture with excipients, or in the form of elixirs or suspensions containing flavouring or colouring agents. They can be injected parenterally, for example, intravenously, intramuscularly or

subcutaneously. For parenteral administration, they are best used in the form of a sterile aqueous solution which may contain other substances, for example, enough salts or glucose to make the solution isotonic with blood.

For oral and parenteral administration to human patients, the daily dosage level of the antifungal compounds of the formula (I) will be from 0.1 to 5 mg/kg (in divided doses) when administered by either the oral or parenteral route. Thus tablets or capsules of the compounds will contain from 5 mg to 0.5 g of active compound for administration singly or two or more at a time as appropriate. The physician in any event will determine the actual dosage which will be most suitable for an individual patient and it will vary with the age, weight and response of the particular patient. The above dosages are exemplary of the average case; there can, of course, be individual instances where higher or lower dosage ranges are merited, and such are within the scope of this invention.

Alternatively, the antifungal compounds of formula (I) can be administered in the form of a suppository or pessary, or they may be applied topically in the form of a lotion, solution, cream, ointment or dusting powder. For example, they can be incorporated into a cream consisting of an aqueous emulsion of polyethylene glycols or liquid paraffin; or they can be incorporated, at a concentration between 1 and 10%, into an ointment consisting of a white wax or white soft paraffin base together with such stabilizers and preservatives as may be required.

The compounds of the formula (I) including their O-esters, O-ethers and salts also have activity against a variety of plant pathogenic fungi, including for example various rusts, mildews and moulds, and the compounds are thus useful for treating plants and seeds to eradicate or prevent such diseases.

The *in vitro* evaluation of the activity of the compounds against plant fungi can be determined by measuring their minimum inhibitory concentrations in the same way as previously described except that the plates are incubated at 30°C for 48 hours or longer before being examined for the presence or absence of growth.

Micro-organisms used in such tests include *Cochliobolus carbonum*, *Pyricularia oryzae*, *Glomerella cingulata*, *Penicillium digitatum*, *Botrytis cinerea* and *Rhizoctonia solani*.

For agricultural and horticultural purposes the compounds and their agriculturally acceptable salts are preferably used in the form of a composition formulated as appropriate to the particular use and purpose desired. Thus the compounds may be applied in the form of dusting powders, or granules, seed dressings, aqueous solutions, dispersions or emulsions, dips, sprays, aerosols or smokes. Compositions may also be applied in the form of dispersible powders, granules or grains, or concentrates for dilution prior to use. Such compositions may contain such conventional carriers, diluents or adjuvants as are known and acceptable in agriculture and horticulture and they are manufactured in accordance with conventional procedures. The compositions may also incorporate other active ingredients, for

example, compounds having herbicidal or insecticidal activity or a further fungicide. The compounds and compositions can be applied in a number of ways, for example they can be applied directly to the plant foliage, stems, branches, seeds or roots or to the soil or other growing medium, and they may be used not only to eradicate disease, but also prophylactically to protect the plants or seeds from attack. The compositions typically contain 0.01 to 95 wt. %, more preferably 0.01 to 1 wt. %, of the active ingredient. For field use, likely application rates of the active ingredient are from 5 to 500 g/10 ares.

The following Examples illustrate the invention. All temperatures are in °C:-

EXAMPLE 1

1-(4-Chlorophenyl)-1-(2,4-difluorophenyl)-2-fluoro-2-(1H-1,2,4-triazol-1-yl)propan-1-ol

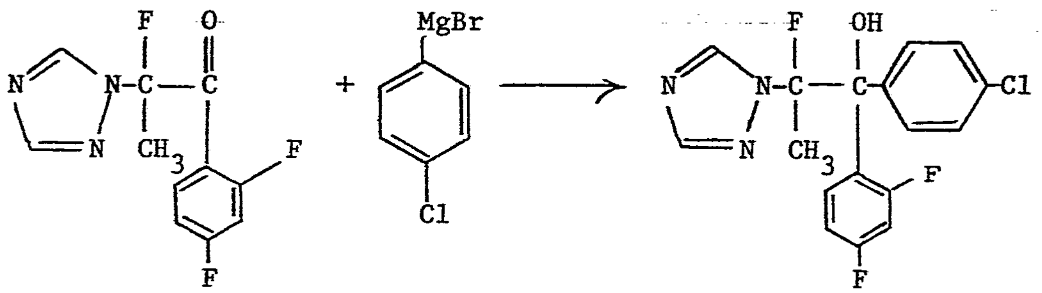

p-Bromochlorobenzene (1.17 g, 6 mM) was added dropwise to magnesium turnings (0.24 g, 10 mM) with stirring under nitrogen in dry ether (20 ml.). A vigorous reaction occurred to give a clear pale brown solution containing excess magnesium turnings. This solution was added dropwise over ten minutes to a solution of 2',4'-difluoro-2-fluoro-2-(1H-1,2,4-triazol-1-yl)propiophenone (0.51 g, 2 mM) in dry ether (20 ml.) with stirring under nitrogen at -70°. The resulting mixture was allowed to warm to room temperature over three hours and was then stirred overnight at room temperature.

The reaction mixture was then treated with a saturated aqueous solution of ammonium chloride (20 ml), the ether layer was separated, washed with brine (20 ml), dried ($MgSO_4$), and evaporated to an oil.

0153803

This oil was flash-chromatographed on silica (Merck, "Kieselgel 60" [Trade Mark]), 40 g, eluting with a mixture of ether and hexane (4:1 by volume). The fractions which contained the desired product (estimated by t.l.c.) were combined and evaporated to a white solid, 0.18 g. This was recrystallised from ethyl acetate to give colourless crystals of the title compound, 0.11 g, m.p. 176-179°, 15% yield.

The N.M.R. spectrum of the product indicated the presence of two diastereomers in approximately equal amounts. These were not separated.

Analysis %:-

| | |
|---|---|
| Found: | C,55.4; H,3.7; N,11.5; |
| Calculated for $C_{17}H_{13}N_3OF_3Cl$: | C,55.5; H,3.6; N,11.4. |

## EXAMPLES 2 - 5

The following compounds were prepared similarly to Example 1 from a ketone of the formula:

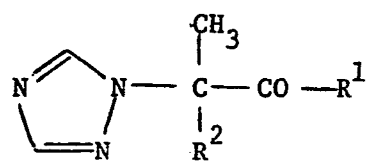

and

Grignard reagent R MgBr to give:-

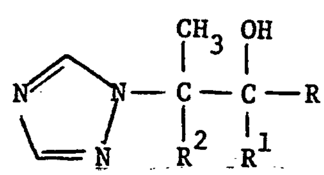

| Example No. | $R^2$ | $R^1$ | R | m.p. (°C) | Analysis % (Theoretical in brackets) C | H | N |
|---|---|---|---|---|---|---|---|
| 2 | H | 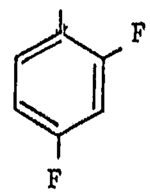 | 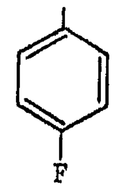 | 125-126 | 61.3<br>(61.2 | 4.2<br>4.3 | 12.6<br>12.6) |
| 3 | $CH_3$ | 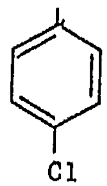 | 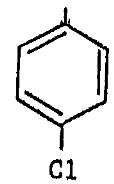 | 164-165 | 59.6<br>(59.7 | 4.8<br>4.8 | 11.8<br>11.6) |
| 4 | $CH_3$ | 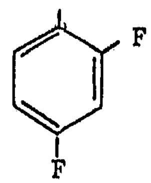 | 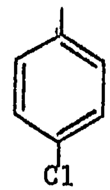 | 159-161 | 59.6<br>(59.4 | 4.5<br>4.4 | 11.7<br>11.6) |
| 5 | $CH_3$ | 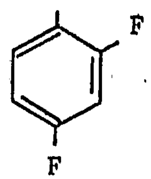 | 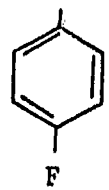 | 146-148 | 62.2<br>(62.2 | 4.7<br>4.6 | 12.2<br>12.1) |

In Examples 2, 3 and 4, the chromatography eluents were, respectively, ether, ether/hexane 9:1, and ether. No chromatography was required in Example 5. The recrystallisation solvent used in Examples 2 to 5 was ethyl acetate containing, respectively, 50%, 30%, 30% and 40% (by volume) hexane. The product of Example 2, as isolated, was one diastereomer only.

## EXAMPLE 6

1,1-Bis(4-fluorophenyl)-2-methyl-2-(1H-1,2,4-triazol-1-yl)propan-1-ol, m.p. 167-9°, was prepared using similar reaction conditions to Example 1 from ethyl 2-(1H-1,2,4-triazol-1-yl)isobutyrate and two equivalents of the Grignard reagent prepared from the reaction of magnesium turnings with p-bromofluorobenzene. The eluent used in the chromatography was ethyl acetate/hexane 4:6 and the recrystallisation solvent was ethyl acetate containing 20% hexane.

Analysis %:-

| | |
|---|---|
| Found: | C,65.7; H,5.2; N,12.8. |
| Calculated for $C_{18}H_{17}N_3OF_2$: | C,65.7; H.5.2; N,12.8. |

## EXAMPLE 7

1-(4-Chlorophenyl)-1-(2,4-difluorophenyl)-2-fluoro-2-(1H-1,2,4-triazol-1-yl)-ethanol

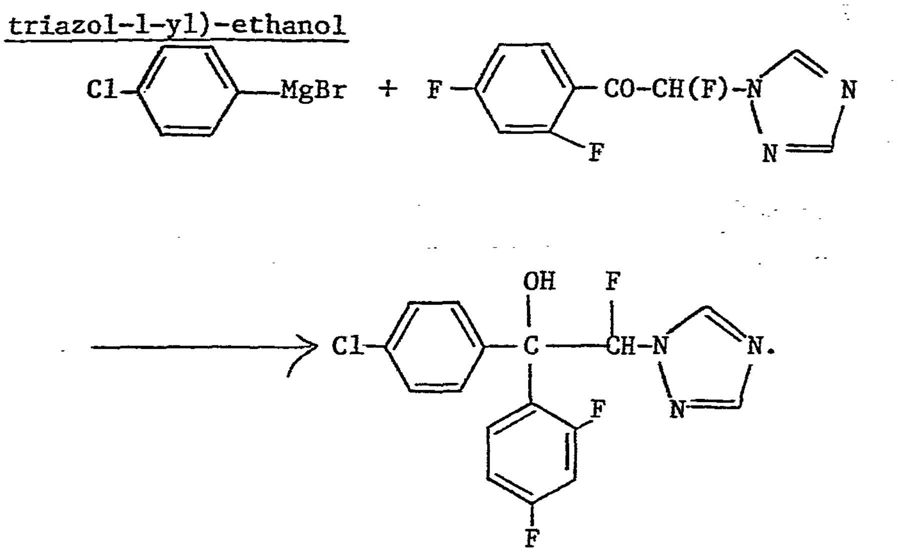

p-Chlorobromobenzene (660 mg) was added to magnesium turnings (129 mg) in dry diethyl ether (50 ml) and the mixture was heated under reflux for 1½ hours. The resultant Grignard reagent was added dropwise to a solution of 2-(1H-1,2,4-triazol-1-yl)-2,2',4'-trifluoroacetophenone (300 mg) in dry diethyl ether (20 ml) whilst stirring at -70° under nitrogen. The reaction mixture was then allowed to warm to room temperature over 1½ hours and heated under reflux for 2 hours. Saturated aqueous ammonium chloride solution (20 ml) was added and the mixture was extracted with diethyl ether (3 x 100 ml). The organic extracts were combined and dried over magnesium sulphate, filtered, and evaporated to a yellow oil which was then chromatographed on silica eluting with a mixture of diethyl ether and n-hexane (4:1) to give, after collection and evaporation of appropriate fractions, diastereomers I (57 mg) and II (45 mg), m.p.'s 199-201° and 146-8° respectively.

Analysis %:-

Found: C,54.3; H,3.2; N,11.8 and

C,54.25; H,3.3; N,12.1, respectively

Required for $C_{16}H_{11}ClF_3N_3O$: C,54.3; H,3.1; N,11.9.

EXAMPLES 8 TO 11

The following compounds were prepared similarly to Example 7 from an acetophenone of the formula R.CO.CH(F). 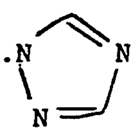 and a Grignard reagent of the formula $R^1$.MgHal. In the case of Example 10, only one diastereomer was isolated.

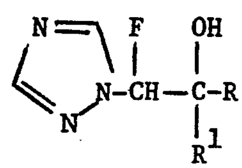

| Example No. | R | $R^1$ | Hal | m.p. (°C) | Analysis % (or n.m.r.) (Theoretical in brackets) C H N |
|---|---|---|---|---|---|
| 8 | 4-Fluorophenyl | 4-Fluorophenyl | Br | 155-6° | 59.8 3.9 13.1<br>(60.2 3.8 13.2) |
| 9 Diastereomer (I) | 4-Fluorophenyl | 2,4-Dichlorophenyl | I | 191-3° | 51.9 3.1 10.9 |
| 9 Diastereomer (II) | 4-Fluorophenyl | 2,4-Dichlorophenyl | I | 163-165° | 51.9 3.0 11.4<br>(51.9 3.0 11.4) |
| 10 | 2,4-Dichlorophenyl | 4-Chlorophenyl | Br | 164-6° | 49.6 3.0 11.5<br>(49.7 2.9 10.9) |
| 11 | 2,4-Difluorophenyl | 2,4-Difluorophenyl | Br | 149-151° | <u>N.m.r.</u> ($CDCl_3$), $\delta$ = 6.4, s, (1H); 7.2 m, (5H); 7.8, m, (2H); 7.85, s, (1H); 8.5, s, (1H). Mass spec., $(M+1)^{\oplus}$ = 356. |

EXAMPLE 12

Resolution of the product of Example 8

The product from Example 8 was reacted with R-(-)-1-(1-naphthyl)ethyl isocyanate to produce two diastereomeric carbamates, which were separated chromatographically. Each carbamate was then subjected to cleavage by treatment with lithium aluminium hydride to generate the optically active forms of the product of Example 8.

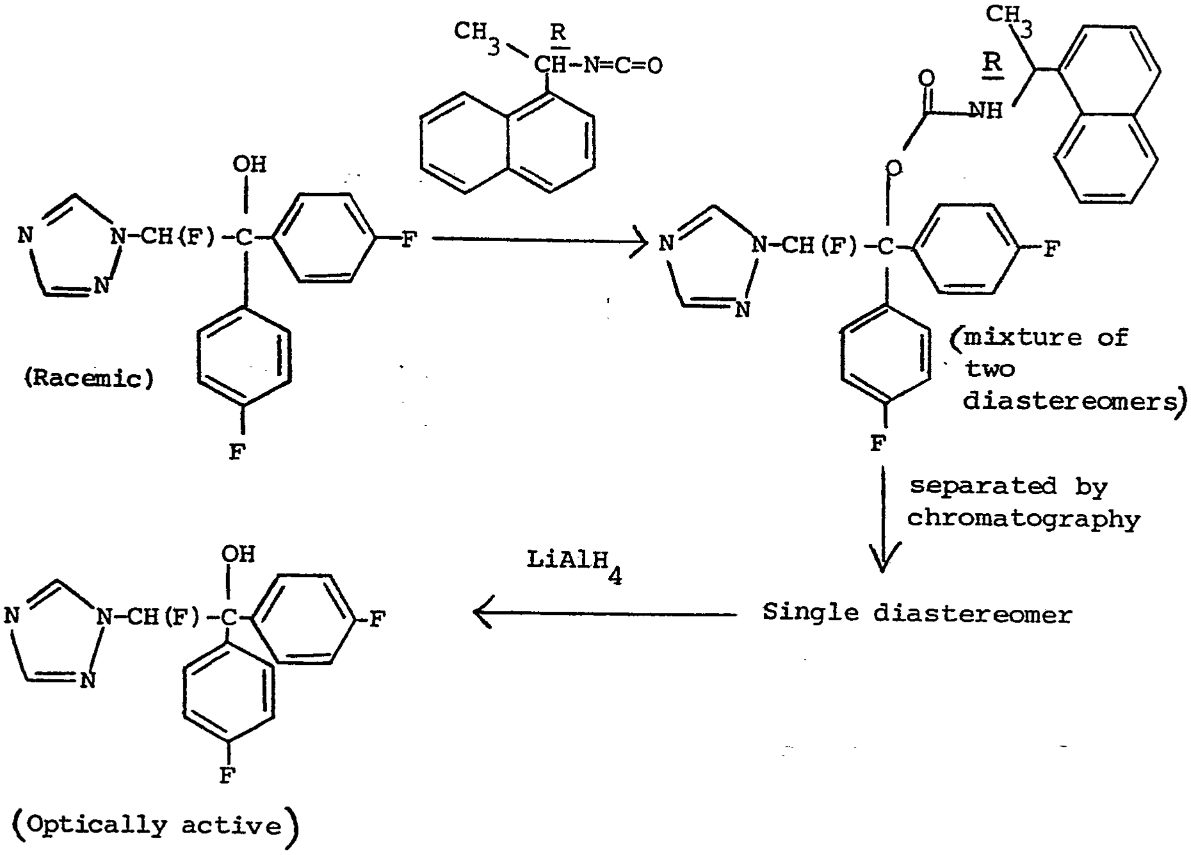


1. Preparation and separation of diastereomeric carbamates

2-Fluoro-1,1-di-(4-fluorophenyl)-2-(1,2,4-triazol-1-yl)ethanol, 1.9 g, R-(-)-1-(1-naphthyl)ethyl isocyanate, 2.6 g, and bis(tri-n-butyl tin)oxide, 120 mg, were heated under reflux in dry tetrahydrofuran (8 ml) in an atmosphere of nitrogen for 48 hours.

The reaction mixture was allowed to cool, poured onto ice (50 g), extracted with ethyl acetate (2 x 50 ml), the organic layer dried ($MgSO_4$), and evaporated to a gum, 5.2 g.

The gum was subjected to repeated flash chromatography on silica (300 g), eluting with a mixture of ethyl acetate:hexane:diethylamine, 50:50:1.5. After one chromatography, the majority of the faster running diastereomer was obtained pure, and about half of the slower running diastereomer.

The remainder of the slower running diastereomer was still contaminated with starting material. It was discovered that a good separation of this diastereomer from starting material could be achieved using a silica column deactivated by standing overnight in the elution mixture i.e. 50:50:1.5, ethylacetate:hexane:diethylamine. Thus after two columns, the majority of each diastereomer was obtained free from the other, but still containing other small impurities. Each diastereomer was subjected to a further flash chromatography using the same eluent as before to provide pure material, faster running diastereomer, (a), 1.2 g, slower running diastereomer (b), 1.1 g, as glasses. The diastereomers were characterised by reduction back to the original alcohol with lithium aluminium hydride (vide infra), and by NMR spectrum, which clearly showed the presence of a triazole proton at 8.58 and a methyl doublet at 1.6 $\delta$, J 7Hz.

0153803

2. Reductive cleavage of diastereomers to optically active alcohols

Diastereomer (a) (990 mg) was stirred at 0° in a mixture of ether (25 ml) and tetrahydrofuran (15 ml) whilst lithium aluminium hydride (45 mg) was added in portions over 40 minutes. After stirring for a further 30 minutes, water was cautiously added, dropwise at first (20 ml). The reaction mixture was then extracted with ethyl acetate (3 x 30 ml), the organic extracts dried ($MgSO_4$) and evaporated to a semi-solid, 990 mg.

Flash chromatography on silica (250 g), eluting with ether containing 1% diethylamine gave the product as a white solid, 560 mg. This material was rechromatographed on silica (250 g) eluting with methylene chloride:methanol:diethylamine, 96:4:1 to remove trace impurities. Recrystallization from ethyl acetate/cyclohexane gave colourless crystals, 480 mg, m.p. 154-155°, $[\alpha]_{589}^{25^\circ}$-141.8° (methanol).

Analysis %:-

| | |
|---|---|
| Found: | C,60.2; H,3.8; N,13.2; |
| $C_{16}H_{12}F_3N_3O$ requires: | C,60.2; H,3.8; N,13.2%. |

Diastereomer (b) was cleaved in an identical manner to give colourless crystals m.p. 151-154°, $[\alpha]_{589}^{25^\circ}$ + 141.5° (methanol).

Analysis %:-

| | |
|---|---|
| Found: | C,59.9; H,3.7; N,12.9; |
| $C_{16}H_{12}F_3N_3O$ requires: | C,60.2; H,3.8; N,13.2. |

The following Preparations, in which all temperatures are in °C, illustrate the preparation of certain starting materials:-

PREPARATION 1

2',4'-Difluoro-2-methyl-2-(1H-1,2,4-triazol-1-yl)propiophenone

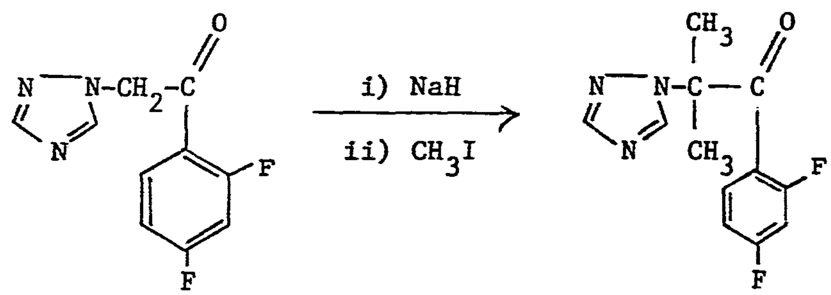


A stirred solution of 2',4'-difluoro-2-(1H-1,2,4-triazol-1-yl)acetophenone (3.7 g, 16.6 m.Mole) in tetrahydrofuran (70 ml) was cooled to 5°, when sodium hydride as a 50% dispersion in oil (1.58 g of said dispersion, which contains 33.2 m.Mole of sodium hydride) was added. Thirty minutes later methyl iodide (5.2 g, 36.5 m.Mole) was added over a five minute period. Stirring was continued for 20 hours at room temperature.

The mixture was then poured into ice-water (150 ml) and extracted with ethyl acetate (3 x 50 ml). The organic extracts were combined, washed with water (3 x 20 ml), and dried over anhydrous magnesium sulphate. Evaporation gave an impure solid, weight 4.6 g.

Purification was carried out by flash column chromatography under slight pressure (2 p.s.i.) on a Merck "Kieselgel 60" 230-400 mesh silica column, eluting with ether.

The appropriate fractions, on evaporation, gave a solid which was recrystallised from cyclohexane/n-pentane giving the pure title compound, 1.5 g, m.p. 45-47° (36.3% yield).

Analysis %

| | |
|---|---|
| Calculated for $C_{12}H_{11}F_2N_3O$: | C,57.4; H,4.4; N,16.7 |
| Found: | C,57.6; H,4.1; N,16.4. |

N.m.r., i.r. and mass spectral data for the product were consistent with the stated structure.

PREPARATION 2

4'-Chloro-2-methyl-2-(1H-1,2,4-triazol-1-yl)propiophenone, m.p. 118-9°, was prepared similarly to Preparation 1 starting from 4'-chloro-2-(1H-1,2,4-triazol-1-yl)acetophenone, sodium hydride and methyl iodide.

Analysis %:-

| | |
|---|---|
| Found: | C,57.8; H,4.9; N,16.9; |
| Calculated for $C_{12}H_{12}ClN_3O$: | C,57.7; H,4.8; N,16.8. |

PREPARATION 3

2',4'-Difluoro-2-(1H-1,2,4-triazol-1-yl)propiophenone hydrochloride

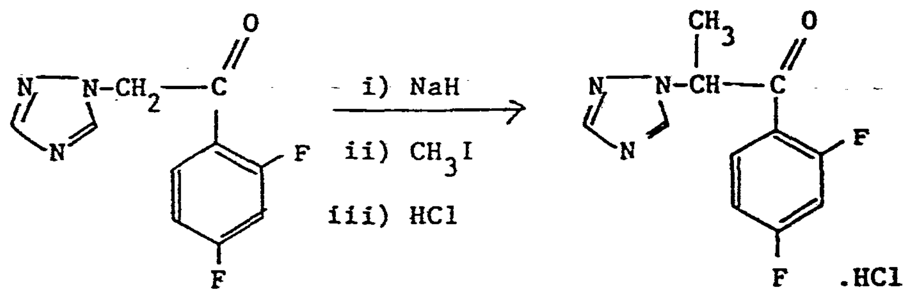


A stirred solution of 2',4'-difluoro-2-(1H-1,2,4-triazol-1-yl)acetophenone (4 g, 17.9 m.Mole) in tetrahydrofuran (75 ml) was cooled to 5°, when sodium hydride as a 50% dispersion in oil (0.86 g of said dispersion, which contains 17.9 m.Mole of a sodium hydride) was added. Twenty minutes later methyl iodide (2.8 g, 19.7 m.Mole) was added over a five minute period. Stirring was continued for 19 hours at room temperature. The mixture was then poured into ice-water (100 ml) and extracted with methylene chloride (3 x 30 ml). The combined organic extracts were washed with water (3 x 40 ml) and dried over anhydrous magnesium sulphate. On evaporation an oil was obtained, weight 4.7 g. Purification was carried out by flash column chromatography under slight pressure (2 p.s.i.) on a Merck "Kieselgel 60" 230-400 mesh silica column, eluting with ether. The appropriate combined fractions were reduced in volume (to 50 ml) by evaporation and then treated with gaseous hydrogen chloride. The resulting

hydrochloride salt was filtered off and recrystallised from isopropanol, giving the pure title compound, 1.66 g, m.p. 147-150°, as fine crystals (33.9% yield).

Analysis %:-

| | |
|---|---|
| Calculated for $C_{11}H_9F_2N_3O.HCl$: | C,48.3; H,3.7; N,15.4 |
| Found: | C,48.1; H,3.5; N,15.7. |

N.m.r., i.r. and mass spectral data for the product were consistant with the stated structure.

PREPARATION 4

Preparation of ethyl 2-(1H-1,2,4-triazol-1-yl)isobutyrate

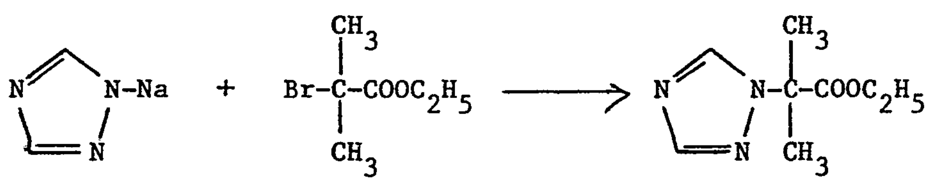

Sodium hydride (12 g of 60% suspension in oil) was stirred at 0° in dry dimethylformamide whilst a solution of 1,2,4-triazole (21 g) in dry dimethylformamide was added. Stirring was continued for 15 minutes at 0°, then ethyl 2-bromoisobutyrate (58.5 g) in dry dimethylformamide (50 ml) was added. When the exothermic reaction had subsided, the reaction mixture was heated at 60-65° for 16 hours. The cooled solution was then filtered, the filtrate evaporated under reduced pressure, and the residual dimethylformamide azeotropically removed by co-distillation with xylene under reduced pressure. The residue was distilled under vacuum to give a colourless oil, b.p. 98° at 0.4 mm Hg, 41.8 g. This material was then flash-chromatographed on silica, eluting with ethyl acetate, to give the title compound as a colourless oil, 29 g.

This material was used without further purification.

N.M.R. ($CDCl_3$) $\delta$ = 1.2, t, J = 7 Hz, 3H; 1.85, s, 6H; 4.17, q, J = 7 Hz, 2H; 7.95, s, 1H; 8.27, s, 1H.

PREPARATION 5

Preparation of 2-(1H-1,2,4-triazol-1-yl)-2,2',4'-trifluoropropiophenone

2',4'-Difluoro-2-(1H-1,2,4-triazol-1-yl)propiophenone (free base, 2.0 g) was stirred at room temperature in dry tetrahydrofuran (20 ml) whilst sodium hydride (0.44 g, 1.3 equiv. of a 60% dispersion in oil) was added. When gas evolution had ceased, a clear solution was obtained, which was exposed to an atmosphere of perchloryl fluoride until no more was absorbed. The solvent was removed under reduced pressure and the residue was carefully treated with ether (50 ml) which had been saturated with water so as to destroy any remaining sodium hydride in the residue. The ether solution was then carefully decanted from the inorganic solids. The remaining solids were washed with dry ether (20 ml), and this ether was decanted and added to the original ether

extract. The ether was then evaporated to give the title compound as a pale yellow oil, 2.0 g, which was used directly without further purification.

N.M.R. ($CDCl_3$): $\delta$ = 8.54, 1H, s; 8.0, 1H, s; 7.8, 1H, m; 6.9, 2H, m; 2.27, 3H, d, J = 19 Hz.

PREPARATION 6

(i) 2,2',4'-Trifluoroacetophenone

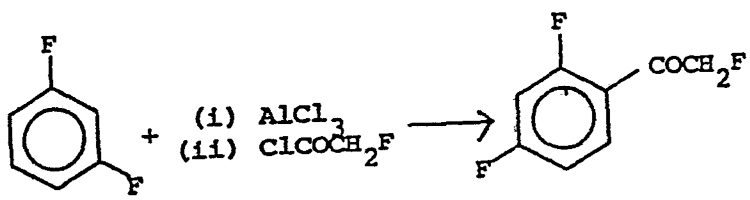


1,3-Difluorobenzene (2.02 g; 17.7 mM) and anhydrous aluminium chloride (2.60 g; 19.47 mM) were stirred together under dry nitrogen at room temperature. A solution of fluoroacetyl chloride (1.71 g; 17.7 mM) in anhydrous methylene chloride (2 ml) was added over 30 minutes. The mixture was then warmed at about 50° for 3 hours. On cooling methylene chloride (40 ml) was added and the mixture was poured onto ice. The methylene chloride layer was separated, dried over magnesium sulphate and evaporated to give a white solid. This material was chromatographed on a column of silica eluting with a mixture of hexane/methylene chloride (65:35 by volume). The product-containing fractions were collected. The title compound crystallised on evaporation of

these fractions and was dried in a vacuum desiccator to a colourless crystalline solid (1.12 g; 36% yield), melting point 57-8°.

Analysis %:-

| | |
|---|---|
| Found: | C,55.0; H,2.8; |
| Required for $C_8H_5F_3O$: | C,55.2; H,2.9. |

(ii) 2,2',4'-Trifluoro-2-chloroacetophenone

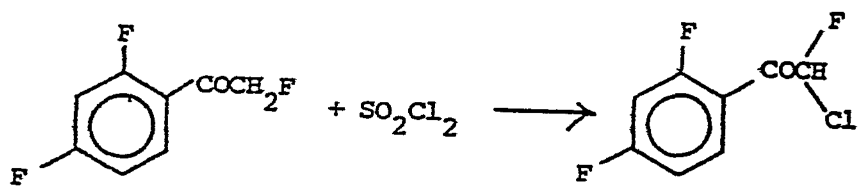


A solution of 2,2',4'-trifluoroacetophenone (1.60 g; 9.2 mM) in sulphuryl chloride (4 ml) was heated at 75° for 18 hours. The mixture was then cooled and iced-water (40 ml) was added. The product was extracted into ether (80 ml), which was washed with water and aqueous sodium bicarbonate solution, and then dried over magnesium sulphate. Evaporation yielded the desired product as a colourless, intensely lachrymatory liquid (2.0 g; 100%).

This liquid had N.M.R. and I.R. spectra consistent with the desired structure and was used directly in the next stage.

[N.m.r. ($CDCl_3$): $\delta$ = 8.05(m,1H); 6.95(m,2H); 6.87(d,1H,J=51)
I.R. (KBr), $-\underset{\underset{O}{\|}}{C}-$ at 1710 $cm^{-1}$.]

(iii) 2-(1H-1,2,4-Triazol-1-yl)-2,2',4'-trifluoroacetophenone

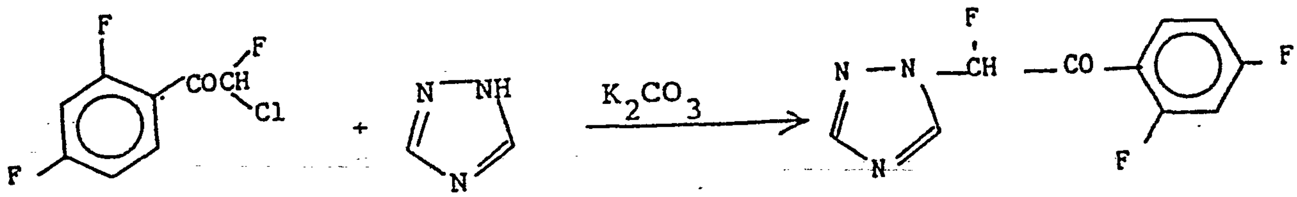


A mixture of 1,2,4-triazole (1.25 g); 18mM) and anhydrous potassium carbonate (2.0 g; 14.5 mM) in anhydrous tetrahydrofuran (20 ml) was stirred at the reflux temperature. A solution of 2,2'4'-trifluoro-2-chloroacetophenone (1.5 g; 7.19 mM) in tetrahydrofuran (10 ml) was then added over a ten minute period. The mixture was refluxed for 2 hours and then stood overnight at room temperature. Water (50 ml) was added and the mixture was extracted with methylene chloride (2 x 100 ml). The combined organic extracts were dried over magnesium sulphate and evaporated to yield a gum. Chromatography on a column of silica, eluting with a mixture of ethylacetate/hexane/diethylamine (ratio 70:30:3 by volume) yielded, after evaporation of appropriate fractions, the desired product as a gum, (130 mg; 7.5%). The n.m.r. was consistent with the desired structure.

The compound was characterised as the methanesulphonic acid salt, m.p. 158-160°, prepared by mixing a solution of the free base in ether/acetone with a solution of the acid in ether/acetone and collecting the precipitated salt.

Analysis %:-

Found: C,39.1; H,3.0; N,12.4;

Calculated for $C_{10}H_6F_3N_3O.CH_4O_3S$: C,39.2; H,3.0; N,12.5.

Preparation 7 (alternative to Preparation 6)

2-(1H-1,2,4-triazol-1-yl)-2,2',4'-trifluoroacetophenone

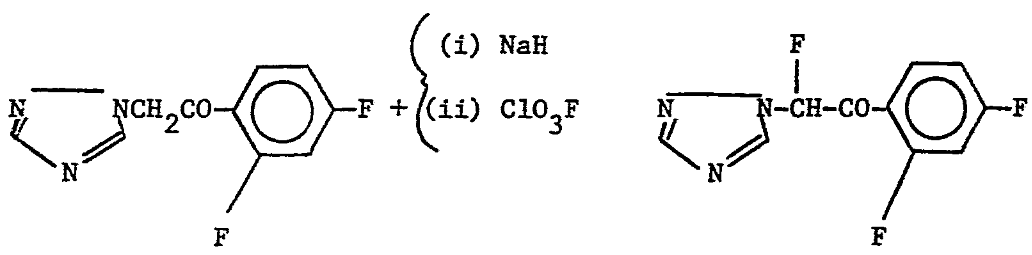


Sodium hydride (285 mg of a 50% dispersion in oil; 5.94 mM of sodium hydride) was washed with ether and dried under nitrogen. Anhydrous tetrahydrofuran was added (15 ml), followed by 2',4'-difluoro-2-(1H-1,2,4-triazol-1-yl)-acetophenone (1.115 g; 5mM) (see U.K. patent application publication no. 2099818A) in portions over a 5 minute period. The resultant brown solution was stirred under an atmosphere of perchloryl fluoride until the theoretical amount had been absorbed. The pale yellow suspension was evaporated and partitioned between water (25 ml) and ethyl acetate (50 ml). The ethyl acetate phase was separated, washed with water, dried over magnesium sulphate and evaporated to give an oil which crystallised (1.21 g).

The product was confirmed by n.m.r. and t.l.c. to be the same as the product of Preparation 6 (iii), free base form, in about 80% purity.

In an alternative method, after evaporation of said suspension, the residue was chromatographed on silica eluting with a mixture of ether and n-hexane (9:1) to yield the title compound.

## PREPARATIONS 8 and 9

The following ketone intermediates were prepared similarly to the process of Preparation 7 from the appropriate acetophenone, sodium hydride and perchloryl fluoride:-

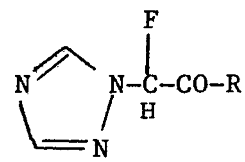

| Preparation No. | R | m.p. (°C) | Analysis % (or n.m.r.) (Theoretical in brackets) C H N |
|---|---|---|---|
| 8 | 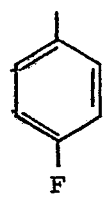 | 98-100 | N.m.r. ($CDCl_3$): $\delta$ = 7.2 (m), 2H; 7.3 (d), 1H, J 48Hz; 8.1 (m), 3H; 8.5 (s), 1H. |
| 9 | 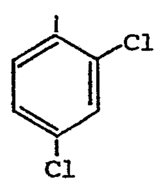 | 64-66 | 43.9 2.2 15.4<br>(43.9 2.2 15.4) |

The starting acetophenones were prepared analogously to the procedure described in GB 2099818A for the preparation of 2',4'-difluoro-2-(1H-1,2,4-triazol-1-yl)acetophenone.

Using the test method described in the text, the following $PD_{50}$ values were obtained vs. Candida albicans in mice:-

| Compound | $PD_{50}$ (mg./kg.) |
|---|---|
| Product of Example 1 | 0.95 |
| Product of Example 2 | < 1.0 |
| Product of Example 3 | < 1.0 |
| Product of Example 4 | 2.3 |
| Product of Example 5 | 2.2 |
| Product of Example 6 | 1.6 |
| Product of Example 7 (diastereomer I) | 1.6 |
| Product of Example 7 (diastereomer II) | 0.3 |
| Product of Example 8 | 0.3 |
| Product of Example 9 (diastereomer I) | 0.3 |
| (diastereomer II) | 0.5 |
| Product of Example 10 | 3.2 |
| Product of Example 11 | <1 |
| Product of Example 12(+) | 0.75 |
| Product of Example 12(-) | 0.2 |

CLAIMS for the Contracting States
BE, CH, DE, FR, GB, IT, LI, LU, NL, SE

1. A compound of the general formula:-

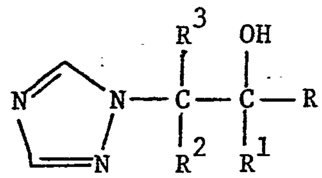

--- (I)

or an O-ester, O-ether or pharmaceutically or agriculturally acceptable salt thereof,

wherein R and $R^1$ are each independently a phenyl group optionally substituted by 1 to 3 substituents each independently selected from F, Cl, Br, I, $CF_3$, $C_1$-$C_4$ alkyl and $C_1$-$C_4$ alkoxy;

$R^2$ is H, $CH_3$ or F;

and $R^3$ is H or $CH_3$;

with the proviso that one of $R^2$ and $R^3$ is other than H.

2. A compound as claimed in claim 1 wherein R and $R^1$ are each independently phenyl substituted by 1 to 3 substituents each independently selected from F, Cl, Br, I and $CF_3$.

3. A compound as claimed in claim 2 wherein R and $R^1$ are each independently 4-fluorophenyl, 4-chlorophenyl, 4-trifluoromethylphenyl, 2-fluorophenyl, 2,4-dichlorophenyl, 2,4-difluorophenyl, 4-chloro-2-fluorophenyl, 2-chloro-4-fluorophenyl, 2,5-difluorophenyl, 2,4,6-trifluorophenyl or 4-bromo-2,5-difluorophenyl.

4. A compound as claimed in claim 3 wherein R and $R^1$ are each independently 4-chlorophenyl, 4-fluorophenyl, 2,4-dichlorophenyl or 2,4-difluorophenyl.

5. A compound as claimed in claim 1 wherein $R^2$ is F and $R^3$ is H.

6. A compound as claimed in claim 1 wherein $R^3$ is $CH_3$ and $R^2$ is H, $CH_3$ or F.

7. A compound as claimed in any one of the preceding claims wherein (a) said O-ester is a $C_2$-$C_4$ alkanoyl or benzoyl ester, said benzoyl group being optionally substituted by 1 or 2 substituents each independently selected from halo, $C_1$-$C_4$ alkyl and $C_1$-$C_4$ alkoxy and (b) said O-ether is a $C_1$-$C_6$ alkyl, $C_2$-$C_4$ alkenyl, $C_2$-$C_4$ alkynyl or benzyl ether, said benzyl group being optionally ring-substituted by 1 or 2 substituents each independently selected from halo, $C_1$-$C_4$ alkyl and $C_1$-$C_4$ alkoxy.

8. A pharmaceutical composition comprising a compound of the formula (I) or an O-ester, O-ether of pharmaceutically acceptable salt thereof as claimed in any one of the preceding claims, together with a pharmaceutically acceptable diluent or carrier.

9. A fungicidal composition for agricultural (including horticultural) use, comprising a compound of the formula (I) or an O-ester, O-ether or agriculturally acceptable salt thereof as claimed in any one of claims 1 to 7, together with an agriculturally acceptable diluent or carrier.

10. A method of treating or preventing a fungal infection on seeds or plants, which contacting said seeds or plants, or the locus thereof, with an antifungally effective amount of a composition as claimed in claim 9.

11. A process for preparing a compound of the formula (I) as claimed in claim 1, characterised by reacting a compound of the formula:-

RMgBr, RMgI or RLi

where R is as defined in claim 1,

with a compound of the formula:-

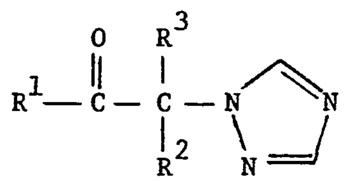

where $R^1$, $R^2$ and $R^3$ are as defined in claim 1, said process being followed by, optionally, one or both of the following:-

(i) conversion of the product into an O-ester or O-ether and

(ii) conversion of the product into a pharmaceutically or agriculturally acceptable salt.

12. A process for preparing a compound of the formula (I) as calimed in claim 1 wherein R and $R^1$ are the same and $R^2$ and $R^3$ are both $CH_3$, characterised by reacting a compound of the formula:-

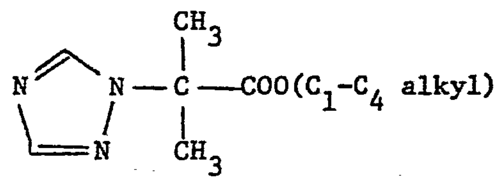

with at least 2 equivalents of a compound of the formula:-

RMgBr, RMgI or RLi,

where R is as defined in claim 1,
said process being followed by, optionally, steps (i) and/or (ii) as defined in claim 11.

13. A compound of the formula (I) or O-ester, O-ether or pharmaceutically acceptable salt thereof as claimed in any one of claims 1 to 7, for use in medicine.

CLAIMS for the Contracting State AT

1. A process for preparing a triazole of the general formula:-

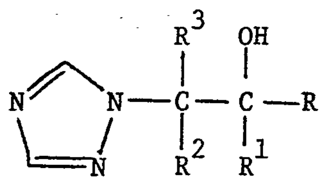

--- (I)

or an O-ester, O-ether or pharmaceutically or agriculturally acceptable salt thereof,

wherein R and $R^1$ are each independently a phenyl group optionally substituted by 1 to 3 substituents each independently selected from F, Cl, Br, I, $CF_3$, $C_1$-$C_4$ alkyl and $C_1$-$C_4$ alkoxy;

$R^2$ is H, $CH_3$ or F;

and $R^3$ is H or $CH_3$;

with the proviso that one of $R^2$ and $R^3$ is other than H;

characterised by reacting a compound of the formula:-

RMgBr, RMgI or RLi

where R is as defined above,

with a ketone of the formula:-

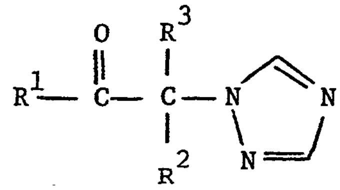

where $R^1$, $R^2$ and $R^3$ are as defined above,

said process being followed by, optionally, one or both of the following:-

(i) conversion of the product into an O-ester or O-ether;

and (ii) conversion of the product into a pharmaceutically or agriculturally acceptable salt.

2. A process as claimed in claim 1, characterised in that R and $R^1$ are each independently phenyl substituted by 1 to 3 substituents each independently selected from F, Cl, Br, I and $CF_3$.

3. A process as claimed in claim 2 characterised in that R and $R^1$ are each independently 4-fluorophenyl, 4-chlorophenyl, 4-trifluoromethylphenyl, 2-fluorophenyl, 2,4-dichlorophenyl, 2,4-difluorophenyl, 4-chloro-2-fluorophenyl, 2-chloro-4-fluorophenyl, 2,5-difluorophenyl, 2,4,6-trifluorphenyl or 4-bromo-2,5-diflourophenyl.

4. A process as claimed in claim 3 characterised in that R and $R^1$ are each independently 4-chlorophenyl, 4-fluorophenyl, 2,4-dichlorophenyl or 2,4-difluorophenyl.

5. A process according to claim 1, characterised in that $R^2$ is F and $R^3$ is $CH_3$.

6. A process according to claim 1, characterised in that $R^2$ is H, $CH_3$ or F and $R^3$ is $CH_3$.

7. A process for preparing a triazole of the formula (I) as defined in claim 1 wherein R and $R^1$ are the same and $R^2$ and $R^3$ are both $CH_3$, characterised by reacting a compound of the formula:-

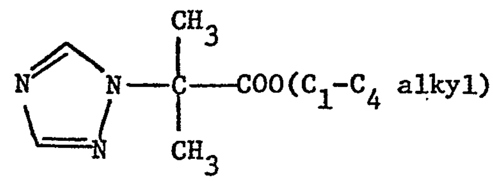

with at least 2 equivalents of a compound of the formula:-

RMgBr, RMgI or RLi

where R is as defined in claim 1,
said process being followed by, optionally, steps (i) and/or (ii) as defined in claim 1.

8. A process according to claim 7, wherein R and $R^1$, which are the same, are 4-fluorophenyl, 4-chlorophenyl, 4-trifluoromethylphenyl, 2-fluorophenyl, 2,4-dichlorophenyl, 2,4-difluorophenyl, 4-chloro-2-fluorophenyl, 2-chloro-4-fluorophenyl, 2,5-difluorophenyl, 2,4,6-trifluorophenyl or 4-bromo-2,5-difluorophenyl.

9. A process for preparing a pharmaceutical composition characterised by mixing a compound of the formula (I) as defined in claim 1, or an O-ether, O-ester or pharmaceutically acceptable salt thereof, with a pharmaceutically acceptable diluent or carrier.

10. A process according to claim 9, characterised in that the compound of the formula (I) or O-ether, O-ester or pharmaceutically acceptable salt thereof has been prepared by a process as claimed in any one of claims 1 to 8.

11. A fungicidal composition for agricultural use, comprising a compound of the formula (I) as defined in claim 1 or an O-ester, O-ether or agriculturally acceptable salt thereof, and an agriculturally acceptable diluent or carrier.